(19) Europäisches Patentamt
European Patent Office
Office européen des brevets

(11) **EP 4 100 520 B1**

(12) **EUROPEAN PATENT SPECIFICATION**

(45) Date of publication and mention
of the grant of the patent:
**27.03.2024  Bulletin 2024/13**

(51) International Patent Classification (IPC):
*C12N 13/00* (2006.01)      *B82Y 5/00* (2011.01)
*C12N 9/88* (2006.01)       *C12Q 1/6883* (2018.01)
*G01N 33/68* (2006.01)      *G06N 10/00* (2022.01)
*G16B 20/00* (2019.01)      *G21K 1/00* (2006.01)
*H04L 9/08* (2006.01)       *H01L 29/00* (2006.01)
*A61N 5/00* (2006.01)       *B01J 8/00* (2006.01)
*B01J 21/00* (2006.01)      *B01J 37/00* (2006.01)
*H04B 5/00* (2024.01)       *H04B 10/00* (2013.01)
*H04B 10/70* (2013.01)      *H02J 50/10* (2016.01)

(21) Application number: **21711951.0**

(22) Date of filing: **22.01.2021**

(52) Cooperative Patent Classification (CPC):
**C12N 13/00; C12Y 402/01011; G06N 10/00;
H04B 10/70; H04L 9/0852;** B82Y 10/00

(86) International application number:
**PCT/IB2021/050480**

(87) International publication number:
**WO 2021/156694 (12.08.2021 Gazette 2021/32)**

(54) **SYSTEM AND METHOD OF TRANSFERRING QUANTUM STATES INFORMATION, ESPECIALLY OF MACROMOLECULAR SYSTEMS**

SYSTEM UND VERFAHREN ZUR ÜBERTRAGUNG VON QUANTUMSTAATEN INFORMATIONEN, INSBESONDERE VON MAKROMOLEKULÄREN SYSTEMEN

SYSTÈME ET PROCÉDÉ DE TRANSFERT D'INFORMATION D'ETATS QUANTUMES, SURTOUT DE SYSTEMES MACROMOLECULAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.02.2020  PL 43285220**

(43) Date of publication of application:
**14.12.2022  Bulletin 2022/50**

(73) Proprietors:
• **Beznosiuk, Jaroslaw**
**44-100 Gliwice (PL)**
• **Wandzel, Czeslaw**
**34-324 Lipowa Twardorzeczka (PL)**

(72) Inventors:
• **Beznosiuk, Jaroslaw**
**44-100 Gliwice (PL)**
• **Wandzel, Czeslaw**
**34-324 Lipowa Twardorzeczka (PL)**

(74) Representative: **JWP Patent & Trademark
Attorneys
Sienna Center, ul. Zelazna 28/30
00-833 Warszawa (PL)**

(56) References cited:
**WO-A1-2016/138399      WO-A2-2009/116025
CN-A- 108 671 406**

• **YANG ZHONGYUE ET AL: "Revealing quantum
mechanical effects in enzyme catalysis with
large-scale electronic structure simulation",
REACTION CHEMISTRY & ENGINEERING, vol. 4,
no. 2, 4 February 2019 (2019-02-04), pages
298-315, XP055799397, DOI:
10.1039/C8RE00213D** Retrieved from the
Internet:
URL:https://pubs.rsc.org/en/content/articl
epdf/2019/re/c8re00213d>

- **SÉRGIO FILIPE ET AL: "Application of quantum mechanics/molecular mechanics methods in the study of enzymatic reaction mechanisms INTRODUCTION", WIRES COMPUT MOL SCI, vol. 7, 1 January 2017 (2017-01-01), XP055799380, DOI: 10.1002/wcms.1281 Retrieved from the Internet: URL:https://onlinelibrary.wiley.com/doi/pd fdirect/10.1002/wcms.1281>**
- **SCHREINER PETER R.: "Quantum Mechanical Tunneling Is Essential to Understanding Chemical Reactivity", TRENDS IN CHEMISTRY, vol. 2, no. 11, 1 November 2020 (2020-11-01), pages 980-989, XP055799394, ISSN: 2589-5974, DOI: 10.1016/j.trechm.2020.08.006 Retrieved from the Internet: URL:https://www.cell.com/trends/chemistry/ pdf/S2589-5974(20)30215-X.pdf>**

**Description**

The object of the invention is a system and a method of transferring quantum states information, in particular of macromolecular systems. The objects of the present invention are applicable in fields of technology that utilise thermodynamic, chemical, biochemical or biological processes, by influencing the quantum and physical state of the medium being polarized, or influencing the course of the process and its parameters. In addition, the objects of the present invention are applicable in telecommunications, cryptography, quantum computers or computer science.

Consumables, such as substrates or catalysts that have characteristic properties, are essential for the proper course of many thermodynamic, chemical, biochemical, biological or metabolic processes in the human body. Usually, these substances are not commercially available, and therefore, they are expensive to obtain and entail high economic burdens. In addition, due to the limited occurrence of a given element in nature, many technological processes must be carried out without them, which significantly impairs the speed and quality of the chemical reaction taking place in the process.

Given the fact that in order to improve the course of many technological processes, such as those used in chemical or biochemical industries, or to improve the quality of various types of media, e.g. fuels or drinking water (in which substances with characteristic properties are necessary), the real problem is not the need to physically deliver a given substance to the system, but only such a change of parameters in the recipient's tank as to be able to control the speed, overall course of a given chemical reaction or affect the quality of a given medium.

Changing the parameters in the recipient's tank may occur, for example, by delivering quantum information about a given consumable to the system. Until now, the transport of quantum information took place only at the level of individual qubits, using the quantum states teleportation from the source of information to its receiver. Solutions based on such a mechanism are used mainly in quantum computer science and quantum cryptology.

It is worth mentioning that, according to recent scientific reports, physical, physicochemical, biochemical or biological changes that take place at the macroscopic level include a simultaneous interaction on quantum and sub-quantum levels, i.e., the information level. These relationships can be illustrated by using the combustion of carbon in oxygen as an example. Physical reactions include thermodynamic transformations of substrates, while physicochemical reactions include the formation of new chemical compounds, such as $CO$ and $CO_2$. At the quantum level, combustion process leads to a number of changes in quantum states of all atoms involved in combustion. As a result, the electrons are strongly excited, which is manifested by strong electromagnetic radiation in the infrared and visible light ranges. Conversely, at the subquantum level, there are significant changes regarding the loss of quantum information of the entire system, manifested by an increase in the number of possible quantum states, and thus an increase in entropy.

In the field of biological thermodynamics, the concept of entropy is also of great importance in describing various processes in living organisms. Biological thermodynamics can be used to describe processes that take place in the body, and which are based on phenomena such as: active and passive transport through biological membranes, filtration and ultrafiltration, formation of bioelectric potentials, energy transformation in metabolic processes, issues of development, evolution, importance of dissipative structures and stationary states far from the state of equilibrium. Living organisms should be treated as systems with a complex and organized structure. Such a system can be described as a measure of the degree of disorganization, i.e., entropy that is a function of the thermodynamic state. As open systems, living organisms exchange entropy with the environment. This entropy is absorbed with food products, and returned with degraded metabolic products and degraded entropy in the form of heat. At the same time, irreversible processes occur in the system, creating entropy. It is believed that during oncogenesis, a precipitation from the stationary state of the system occurs. In such a situation, positive processes, such as metabolism or combustion are increased, resulting in the transmission of entropy to the environment, manifested, for example, by increased heat transfer or fever. Increasing heat exchange is aimed at restoring the correct energy balance, i.e., entropy. As a result, the body may return to its initial state, namely health, or, in the absence of adaptation, death may occur. This situation takes place in various pathological states, but during oncogenesis, a third, disordered and "self-regulating" state can be distinguished. It is a state, in which entropy increases to a critical value and stabilizes in a new state that does not lead to cell death. It is understood that cancer does not age, but rejuvenates, i.e., progresses, especially in the case of malignant cancers (Madej J., Relacje termodynamiczne między nowotworem a organizmem żywiciela, Medycyna Wet. 2000, 56(8), 486:489).

It is evident from the above that in a living organism, the total change in entropy can be positive, negative or equal to zero. In the last case, the system can be defined as remaining in a state of dynamic equilibrium, i.e., in an open stationary state, which is a characteristic of a healthy, mature system. The increase in entropy will mean the exit from the state of dynamic equilibrium, resulting in the death of the body. The development of an organism, its stabilization period, aging and death, are the relatively well-known thermodynamic phenomena. From these data it is evident that the rate of entropy production is a measure of the organism viability. An example of "rejuvenation" of tissue in the thermodynamic or even morphological sense could be cancer tissue, which, by being in constant state of development, gives more heat to the environment, meaning that it is characterized by a greater dissipation of energy than a mature organism. As a result, the source of entropy is greater than the minimum required by the Prigogine principle, which is manifested by a higher speed of processes. Therefore, cancer cells function at a higher level of entropy than normal cells, which also affects the change of their activity. (Madej J., Relacje termodynamiczne między nowotworem a organizmem żywiciela, Medycyna Wet. 2000, 56(8), 486:489).

It is argued that the concept of entropy is of great importance in the description of processes of both inanimate systems and living organisms. Given the effectiveness of those processes, one should therefore strive to reduce the entropy value and transform the system into a state of order that facilitates the course of many important processes.

At the temperature of 0K, the perfect crystal, which is a body without imperfections, has a total entropy equal to 0, because the entropy of the configuration equals 0 (its state can only be implemented in one way (3$^{rd}$ law of thermodynamics), and the entropy of thermal vibrations equals 0 as well. Thus, in a monocrystal at the temperature of 0K, the level of quantum information is the highest, as the probability of finding any atom in a given quantum position and state is equal to 1. As the temperature increases, the number of possible configurations increases as well and therefore, the amount of information decreases, because the probability of finding a given atom in a given location and in a given quantum state is less than 1, and it decreases further as the temperature rises.

The American Patent US6314189B1 discloses a quantum communication method and device using quantum teleportation that ensures the security of the transmitted data and can be used in cryptology. The solution is to transport a quantum state or a single qubit between photons emitted by a laser. For the teleportation of quantum information, three photons generated by the laser are used. First, the first photon is polarized according to the information to be sent. The second and third photon are quantum entangled and arranged in the information transmitter and receiver, respectively. The transmitter measures polarized states of the first and second photon, and the information about them is then sent to the receiver by classical communication. The receiver captures information about the state of the first photon from the third photon, based on the measurement of the third photon and the results sent by the sender.

From the description of the Chinese patent document CN108604316A, a device and method of interaction with quantum information stored in spins is known. In the disclosed invention, the quantum information processor may comprise one or more quantum information donors, which are non-gaseous atoms of chalcogens (group 16 of the periodic table) and one or more optical resonators. In some embodiments, the quantum information processor comprises receptors, used for receiving quantum states information of free electrons in a semiconductor substrate (in this case silicon), which are made of elements in the boron group (group 13 of the periodic table). In some embodiments, a photon is placed in the resonator that can be quantum entangled with the donor, making it possible to use them for quantum teleportation of electron spin information qubit.

The Chinese patent CN1840486A discloses a device for activating and magnetizing water to increase its energy level. The essence of the device is that water is passed through a vertical channel in which the water is saturated with quantum information and can be consumed by the user immediately after passing through the system. Water washes magnets and elements, which are carriers of quantum information, made of quartz crystal or jade and placed inside. The water thus enriched supports the functioning of the cardiovascular system and reduces salt and cholesterol levels in the body.

The technical problem of the present invention is to propose a system and method of transmitting quantum information that will provide the ability to teleport information about the quantum states of solids, liquids, gases, plasma and any mixtures of these states at any distance. It is desirable that the system and method facilitate the transmission of information on the entropy level of the source, catalytic and biocatalytic effects, spin states of electrons, polarisation state of the source, crystal lattice structure and quantum features. Additionally, it is desirable that components of the system for transferring quantum states information are manufactured from widely available materials. It is also desirable that information on quantum states is transmitted to the end medium in a continuous, stable manner and not lost in time.

The first object of the invention is a system for transferring quantum states information, particularly of macromolecular systems, comprising a source medium of quantum information, a transmitter and receiver of quantum states information and an end medium, characterized in that it further comprises a quantum states resonator in contact with an intermediate medium, the transmitter and receiver of quantum states information being at least two pairs of entanglement elements Ia-Ib, IIa-IIb, wherein the first entanglement element Ia of the first pair of entanglement elements Ia-Ib is connected to a quantum states generator in contact with the source medium of quantum information, and the second entanglement element Ib of the first pair of entanglement elements Ia-Ib is connected to a quantum states resonator, and comprises at least one end tank in contact with the end medium, wherein the first entanglement element IIa of at least one second pair of entanglement elements IIa-IIb is connected to a quantum states resonator, and the second entanglement element IIb of at least one second pair of entanglement elements IIa-IIb is connected to at least one end tank.

Preferably, individual pairs of entanglement elements - first Ia-Ib and at least one second IIa-IIb - are quantum entangled such that the first entanglement element Ia of the first pair of entanglement elements Ia-Ib is quantum entangled with the second entanglement element Ib of the first pair of entanglement elements Ia-Ib, and the first entanglement

EP 4 100 520 B1

element IIa of at least one second pair of entanglement elements IIa-IIb is quantum entangled with the second entanglement element IIb of at least one second pair of entanglement elements IIa-IIb, wherein the quantum entanglement of the first pair of entanglement elements Ia-Ib is independent of the quantum entanglement of at least one second pair of entanglement elements IIa-IIb.

Preferably, individual pairs of entanglement elements Ia-Ib, IIa-IIb are made of a material that is a chemical element in the boron group of the periodic table or an element, which on the valence shell of an atom has an odd number of electrons and is in the form of a solid, or a material in which there are covalent bonds between atoms, and which is in the form of a solid.

Preferably, the source medium of the quantum information is a solid, liquid, gas, plasma, or any mixture thereof and/or constitutes a monocrystal formed from a chemical element in the carbon group of the periodic table.

Preferably, the intermediate medium and/or the end medium is a gas, a liquid, a solid, or a mixture thereof.

Preferably, the quantum states resonator and/or at least one end tank is made of polar dielectric.

Preferably, the quantum states resonator and/or at least one end tank is covered with insulation.

The second object of the invention is a method of transferring quantum states information, in particular of macromolecular systems, characterized in that it comprises the steps of:

a) receiving quantum states information from the source medium of quantum information through the first entanglement element Ia of the first pair of entanglement elements Ia-Ib connected to the quantum states generator in contact with the source medium of quantum information;

b) transmitting the quantum states information of the source medium of the quantum information from the first entanglement element Ia of the first pair of entanglement elements Ia-Ib to the second entanglement element Ib of the first pair of entanglement elements Ia-Ib;

c) transmitting the quantum states information from the second entanglement element Ib of the first pair of entanglement elements Ia-Ib to the quantum states resonator connected to the second entanglement element of the first pair of entanglement elements Ia-Ib, and then to the intermediate medium in contact with the quantum states resonator;

d) receiving the quantum states information of the intermediate medium through the first entanglement element IIa of at least one second pair of entanglement elements IIa-IIb connected to the quantum states resonator;

e) transmitting the quantum states information of the intermediate medium from the first entanglement element IIa of at least one second pair of entanglement elements IIa-IIb to the second entanglement element IIb of at least one second pair of entanglement elements IIa-IIb;

f) transmitting the quantum states information of the intermediate medium from the second entanglement element IIb of at least one second pair of entanglement elements IIa-IIb to the end medium in contact with at least one end tank connected to the second entanglement element IIb of at least one second pair of entanglement elements IIa-IIb.

Preferably, individual pairs of entanglement elements - first Ia-Ib and at least one second IIa-IIb - are quantum entangled such that the first entanglement element Ia of the first pair of entanglement elements Ia-Ib is quantum entangled with the second entanglement element Ib of the first pair of entanglement elements Ia-Ib, and the first entanglement element IIa of at least one second pair of entanglement elements IIa-IIb is quantum entangled with the second entanglement element IIb of at least one second pair of entanglement elements IIa-IIb, wherein the quantum entanglement of the first pair of entanglement elements Ia-Ib is independent of the quantum entanglement of at least one second pair of entanglement elements IIa-IIb.

Preferably, individual pairs of entanglement elements Ia-Ib, IIa-IIb are made of a material that is a chemical element in the boron group of the periodic table or an element, which on the valence shell of an atom has an odd number of electrons and is in the form of a solid, or a material in which there are covalent bonds between atoms, and which is in the form of a solid.

Preferably, the source medium of the quantum information is a solid, liquid, gas, plasma, or any mixture thereof.

Preferably, the source medium of quantum information is a monocrystal composed of a chemical element in the carbon group of the periodic table.

Preferably, the intermediate medium and/or the end medium is a gas, a liquid, a solid, or a mixture thereof.

Preferably, the quantum states resonator and/or at least one end tank is made of polar dielectric.

Preferably, the quantum states resonator and/or at least one end tank is covered with insulation.

The system and method of transferring quantum states information, especially of macromolecular systems, provides teleportation, at any distance, of quantum states information regarding solids, liquids, gases, plasma and any mixtures of these states, acting as so-called quantum states generators (QSG), which enable the use of properties of a given medium whenever it is not universally available. Quantum information may be delivered directly to the medium of selected recipient, or directly to substrates involved in thermodynamic, chemical, biochemical and biological processes, etc., thereby guaranteeing a very wide range of applications of the disclosed invention. In addition, quantum information is transmitted from the source medium to the end medium in a continuous, stable manner and is not lost over time.

The transfer of quantum states information (i.e., teleportation) is related to the non-locality of quantum phenomena and the ability of some materials to receive sub-quantum information, i.e., subtle quantum and relativistic effects, during the direct interaction of unpaired conduction electrons from entanglement elements with matter electrons, on the one hand, the source of quantum information (the source medium of quantum information in contact with the quantum states generator), and on the other hand, the recipient of this information, i.e., objects/medium/processes intended for polarization.

In the process of receiving and transmitting subquantum information, apart from teleportation using quantum entanglement, the interaction of electrons with neighbouring charges by exchanging virtual photons plays a fundamental role. This exchange occurs in all electron interactions currently known in quantum physics, such as: spin-orbit, spin-spin, atom nucleus-electron, electron-electron, crystal lattice phonon- conductor electron, spin waves, charge density waves, spin density waves, electrostatic phenomena, Copper electron pairs formation, Hall effects (classic, anomalous, spin, reverse spin, quantum, quantum-spin), spin Nerst effect, polarization of polar dielectrics, and surface plasmon resonance (collective vibrations of free electrons located close to the surface).

In entanglement elements, quantum entanglement, i.e., correlated quantum states, is realized in materials made of elements in group 13 of the periodic table (boran group), such as: aluminium (Al), indium (In), or an element which, on the valence shell of an atom, has an odd number of electrons and is in the form of a solid, or a material in which there are covalent bonds between atoms and which is in the form of a solid. In the formation of covalent bonds between atoms having unfilled electron shells, the bond is formed only with external electrons with wave functions that extend the farthest from the nucleus. In this situation, bound atoms return one electron to the molecular binding orbital, so that they form a pair of electrons with opposite spins. In a covalent bond we are dealing with a large accumulation of electric charge between bound atoms. One requirement for covalent bonding is an adequate distance between atoms forming a molecule or a condensed phase, which must be small enough to ensure that electron orbits of adjacent atoms overlap. As the distance between adjacent atoms increases, there is a significant decrease in the attraction force. It should be noted that a covalent bond is a directional bond.

In one classification of crystal structures, based on Goldschmidt's laws, we distinguish: covalent crystals (for example, silicon, germanium, diamond, grey tin), ionic crystals, metallic and molecular crystals. In carbon, silicon and germanium crystals, a diamond structure, in which each atom is bound to the four most closely adjacent atoms located in the corners of the tetrahedron, can be present. The carbon atom C has valence of 2 and configuration of $1s^2 2s^2 2p^2$. However, it should be noted that the carbon atom forming the compounds is in excited state, characterized by a configuration of $1s^2 2s^1 2p^3$, wherein all electrons 2s and 2p have parallel spins. Generally, states 2s and 2p occur as linear combinations of sp and not in pure form. Angles of 109.5° are present between bonds formed in four directions.

Total saturation of the covalent bond is observed in the following elements:

- group IV (C, Si, Ge) in tetrahedral configuration,

- group V (P, As, Sb) in a three-fold symmetry configuration,

- group VI (Te, Se) in double-axis chain structures.

Type AIIIBV compounds exhibit bonds that are similar to covalent ones. Compounds of this group include, but are not limited to InSb or GaAs. In a InSb molecule, indium has three valence electrons $5s^2 5p^1$, while antimony has five: $5s^2 5p^3$. When one electron p passes from antimony to indium, an additional covalent bond forms, and as a result, four bonds of this kind can be present in the whole compound.

In the case of formation of a covalent bond by two different elements, such a bond is partially ionic in nature and is called a polarized bond. The difference in electronegativity of atoms in this bond is a measure of its ionicity. It should be emphasized that an unpolarised covalent bond comprises a pair of electrons that belongs to both bonding atoms equally, wherein bonds between the same atoms are always unpolarised.

A further group in the classification of crystal structures includes molecular crystals that are organic compounds, excluding ionic compounds, and inorganic compounds (e.g., crystals of nitrogen, oxygen, noble gases, compounds composed of phosphorus and nitrogen or sulphur, metal carbonyls). In molecular crystals, Van der Waals interactions occur between neutral molecules. The following interactions can be distinguished: dipole-dipole between molecules that have permanent dipole moments (energy in the range of 5-8 kcal/mol, e.g., hydrogen bonds) permanent dipole-induced dipole (energy in the range of 1-2 kcal/mol of type e.g., interactions of hydrogen chloride-toluene in crystals of toluene dihydrochloride) or induced dipole-induced dipole (e.g., staking in DNA).

In addition, homodesmic crystals, which are formed by elements and fairly simple chemical compounds that comprise a small number of elements, can be observed. Homodesmic crystals include covalent crystals, ionic crystals, metal crystals and noble gas crystals (van der Waals interactions). Another group includes heterodesmic crystals, which include molecular crystals, and all other crystals with several different chemical bonds.

Quantum entanglement in materials made of the above-mentioned elements is achieved by creating a new crystal lattice, e.g., by thermal method, including annealing at the plasticity temperature of the homogeneous material, so that the existing crystal lattice is destroyed and a new one is created in this place, which will ensure quantum, relativistic and information independence from all historical quantum, relativistic and information interactions. After cooling to ambient temperature, the homogeneous material thus prepared is mechanically divided into any number of entanglement elements which are quantum entangled. Each separated element, i.e., the entanglement element, is then entangled with all entanglement elements extracted from the annealed and homogeneous portion of the material.

Entanglement elements are receivers of quantum information from the source material of quantum information in contact with the quantum states generator. Also, due to quantum entanglement, individual pairs of entanglement elements transfer quantum information about the quantum states of source material of quantum information between each other. Additionally, entanglement elements transmit quantum information to a quantum polarized medium, i.e., to the end medium in contact with the end tank. The end medium (as well as the end tank) are polar dielectrics such as glass, water, etc.

All chemicals, both occurring naturally and obtained artificially in laboratories, can be characterized by a multiform of crystalline forms, referred to as polymorphism. Polymorphism is believed to result from differences in the packaging or conformation of molecules. Crystallization conditions, e.g., solvent, pressure, temperature, time, affect the packaging of molecules in chemical compounds. In addition, the ratios of ionic rays, particle size or shape, or conformational lability favor different packaging. Pseudopolymorphism, in turn, indicates a tendency of a chemical compound to co-crystallize with a solvent.

In some embodiments of the invention, the shell of the quantum states generator (QSG) is made of an entanglement element closely adjacent to the core surface, enabling the interaction at quantum and sub-quantum levels. The larger contact area of these two elements guarantees a faster transmission of quantum information from the source material of quantum information to the entanglement element. The presence of an intermediate tank, a quantum states resonator (QSG), enables the use of multiple independent pairs of entanglement elements for transmitting quantum information and as a result enables transmitting the quantum information to multiple distant recipients at the same time, using only one sample of the quantum information source medium in contact with the quantum states generator (QSG). No exchange of quantum information source medium or entanglement elements is required in the system as they are not used in the process of transmitting quantum states information. In addition, the entanglement elements used in the invention enable safe, fast and cost-effective generation of a pair of quantum entangled macromolecular elements, wherein the material of these entanglement elements (i.e., the element in the boron group of the periodic table) is widely available and has a relatively low price.

In the present invention, the basic characteristics of quantum information transmitted in the process of transmitting quantum states information are:

- information about the entropy level of the source material of quantum information (in contact with the quantum states generator);

- information about catalytic or biocatalytic interaction;

- information about spin states of electrons of the source material of quantum information;

- information about the polarisation status of the source material of quantum information (solid, liquid, gaseous, plasma);

- information about the crystal lattice structure of the source material of quantum information;

- information about the quantum characteristics of atoms, molecules or their alloys, solutions, mixtures of the source material of quantum information.

The solution of the invention is shown in the following embodiments and is illustrated in the drawing, in which fig. 1 shows a system for transferring quantum states information according to the first, second, third and fourth embodiment of the present invention, fig. 2 shows a system for transferring quantum states information according to a fifth embodiment of the present invention, fig. 3 is a schematic diagram of the construction of a quantum states generator, fig. 4 shows a schematic diagram of the construction of a quantum states resonator, fig. 5 shows a schematic diagram of the construction of the end tank, fig. 6 shows a photo of an electrophoretic test for the glycation products of enolase with methylsiloxane.

Example 1

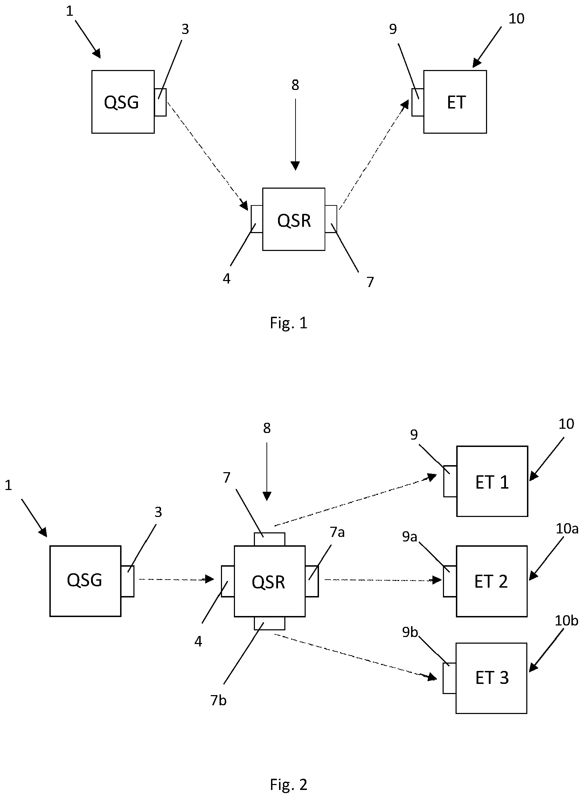

The system for transferring quantum states information, especially macromolecular systems according to a first embodiment of the present invention, is shown schematically in fig. 1. In general, the system for transferring quantum states information comprises a quantum states generator 1 (QSG), a quantum states resonator 8 (QSR) made of polar dielectric 5, such as quartz glass, and an end tank 10 (ET), also made of polar dielectric 5, such as quartz glass. The system also comprises two pairs, Ia-Ib and IIa-IIb, of entanglement elements 3, 4, 7, 9 that are quantum entangled and connected to a quantum states generator 1 (QSG), a quantum states resonator 8 (QSR), and an end tank 10 (ET) in a following manner:

i) Quantum states generator 1 (QST) - first entanglement element 3 Ia of the first pair of entanglement elements Ia-Ib;
ii) Second entanglement element 4 Ib of the first pair of entanglement elements Ia-Ib - quantum states resonator 8 (QSR);
iii) Quantum states resonator 8 (QSR) - first entanglement element 7 IIa of the second pair of entanglement elements IIa-IIb;
iv) Second entanglement element 9 IIb of the second pair of entanglement elements IIa-IIb - end tank 10 (ET).

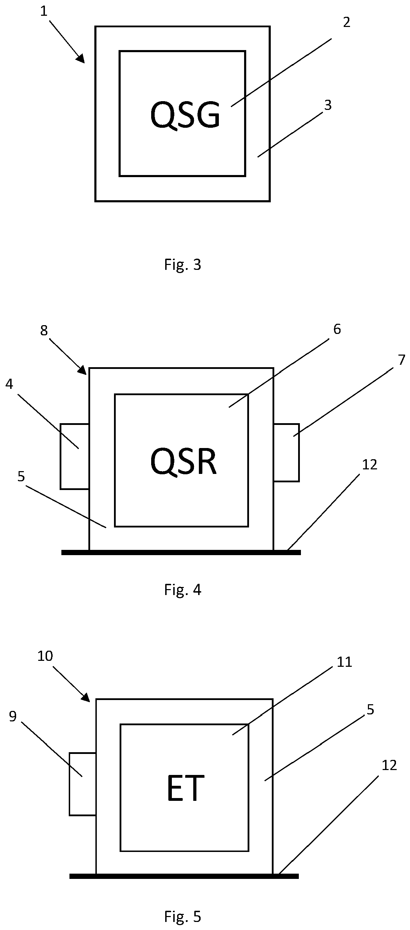

In the present embodiment, the source medium 2 of quantum information is a silicon monocrystal, and in both the intermediate medium 6 and the end medium 11, it is water. The silicon monocrystal in the form of a homogeneous solid constitutes the core of the quantum states generator 1 and is fully coated with entanglement element 3, which constitutes the shell of the quantum states generator 1. Monocrystals are characterized by a very low level of entropy, which is close to zero, and this exact quantum information is transferred to the end medium 11. Fig. 3 provides a schematic illustration of the construction of the quantum states generator 1.

Entanglement elements 3, 4, 7, 9 are made of aluminium (in the boron group) and are quantum entangled with each other: the first entanglement element 3 Ia of the first pair of entanglement elements Ia-Ib with the second entanglement element 4 Ib of the first pair of entanglement elements Ia-Ib and the first entanglement element 7 IIa of the second pair of entanglement elements IIa-IIb with the second entanglement element 9 IIb of the second pair of entanglement elements IIa-IIb.

In the present embodiment, the quantum states resonator 8 is a quartz glass (polar dielectric) tank and is filled with intermediate medium 6, in this example with water. The second entanglement element 4 Ib of the first pair of entanglement elements Ia-Ib and the first entanglement element 7 IIa of the second pair of entanglement elements IIa-IIb are attached to the wall of the quantum states resonator 8 (water tank). Furthermore, in the present embodiment, the quantum states resonator 8 (water tank) is disposed on the insulation 12, which is a wooden washer. The schematic construction of the quantum states resonator 8 is shown in fig. 4.

In the present embodiment, the end tank 10 is a quartz glass (polar dielectric) tank and is filled with the end medium 11, in this example with water. A second entanglement element 9 IIb of the second pair of entanglement elements IIa-IIb is attached to the wall of the end tank 10. Furthermore, in the present embodiment of the invention, the end tank 10 is disposed on the insulation 12, which is a wooden washer. The schematic construction of the end tank 10 is shown in fig. 5.

Quantum states information of the source medium 2 is transmitted by quantum teleportation to the intermediate medium 6 located in the quantum states resonator 8 (QSR), and then to the end medium 11 located in the end tank 10 (ET). As a result of this process, the water placed in the end tank 10 (ET) converts to a coherent state and obtains the quantum states information of the source medium 2, and in the present embodiment, it is information about very low entropy.

Example 2

The system for transferring quantum states information according to the second embodiment of the present invention comprises components substantially similar to those of the system for transferring quantum states information according to the first embodiment of the present invention. The diagram of the second embodiment of the system for transferring quantum states information is analogous to the diagram of the first embodiment shown in fig. 1. For clarity of the present disclosure, a description of concurrent elements of system construction is omitted.

Unlike the first embodiment, the system for transferring quantum states information according to the second embodiment comprises a quantum states generator 1 (QSG) with a source medium 2 of quantum information, which is a silver monocrystal. The intermediate medium 6 of the quantum states resonator 8 (QSR) is a mixture of methanol and air, and the end tank 10 (ET) is a reactor in which the process takes place, requiring a reduction in the entropy of the system. In the present embodiment, an oxidation reaction takes place in the reactor during the production of formaldehyde from methanol and air. Silver is a catalyst for oxidation reaction. Through quantum teleportation, quantum information about the low entropy of the source medium 2 of quantum information and about the catalytic properties of silver is transmitted to the end tank 10 (ET) and therefore, the level of entropy in the recipient reactor decreases, and the expected chemical reaction occurs, i.e., there is an effective oxidation reaction resulting in the formation of formaldehyde.

Example 3

The system for transferring quantum states information according to a third embodiment of the present invention comprises components substantially identical to those of the system for transferring quantum states information according to the first and the second embodiment of the present invention. The diagram of the third embodiment is analogous to the diagram of the first and second embodiment shown in fig. 1. For clarity of the present disclosure, a description of concurrent elements of system construction is omitted.

Unlike the first and second embodiment, the system for transferring quantum states information of the third embodiment comprises entanglement elements 3, 4, 7, 9 made of another element in the boron group of the periodic table, in this example of boron. 99.999% pure boron tiles are commercially available from American Elements, Los Angeles, USA. The source medium 2 is, as in the third example, a silver monocrystal, the intermediate medium 6 is a mixture of air and ethylene, while the end tank 10 (ET) is a recipient reactor in which ethylene is converted into ethylene oxide, which, following hydrolysis, yields ethyl glycol used to produce polyester. Silver is the catalyst in ethylene oxidation reaction. Entanglement elements 3, 4, 7, 9 are quantum entangled with each other, which guarantees the possibility of transferring quantum states information from source medium 2 (silver monocrystal) to quantum states resonator 8 (QSR) by quantum teleportation. Also, in this example, information about the low entropy of the source is transferred to the end tank 10 (ET), resulting in a decrease in entropy in the reactor system and information about the presence of silver in the system, inducing the occurrence of the ethylene oxidation reaction.

Example 4

The system for transferring quantum states information according to a fourth embodiment of the present invention comprises components substantially identical to those of the system for transferring quantum states information according to the first embodiment of the present invention. The diagram of the fourth embodiment is analogous to the diagram of the first, second and third embodiment shown in fig. 1. For clarity of the present disclosure, a description of concurrent elements of system construction is omitted.

Unlike the first, second and third embodiment, the system for transferring quantum states information of the fourth embodiment comprises a source medium 2 in the form of nitrogen oxide (II), NO, which is a catalyst in the sulphur oxide (VI), $SO_3$, formation reaction. In order to maintain the homogeneity of phases of all media used in teleportation of quantum states information and participating in the reaction, intermediate medium 6 located in the quantum states resonator 8 (QSR) is a gaseous medium, air. The quantum states resonator 8 (QSR) is located on an insulation 12 made of material that does not transfer quantum information, in the present embodiment it is wood. However, the end tank 10 (ET) is a reactor that is also located on the insulation layer 12. In the end tank 10 (ET), there are gaseous reaction substrates, i.e., sulphur (IV) oxide, SOz, and oxygen, $O_2$. Quantum information about the catalytic properties of the source medium 2 is transmitted to the reactor, which ensures the correct reaction rate without the need to physically supply a catalyst in the form of nitrogen oxide (II), NO, to the end tank 10 (ET).

Example 5

The system for transferring quantum states information according to the fifth embodiment of the present invention is shown schematically in fig. 2. The system for transferring information comprises, as in examples 1-4, a quantum states generator 1 (QSG) and a quantum states resonator 8 (QSR).

Unlike embodiments 1-4, the system for transferring quantum states information according to the fifth embodiment comprises four pairs of independent entanglement elements 3, 4, 7, 7a, 7b, 9, 9a, 9b, which enable the simultaneous transmission of quantum information regarding the catalytic properties of source medium 2 to three recipients. The system comprises three separate end tanks 10, 10a, 10b (ET1, ET2, ET3), so that the desired reaction can be carried out in three different recipients. Four entanglement elements 4, 7, 7a, 7b of independent entanglement element pairs are connected to the quantum states resonator 8 (QSR). One of them is a second entanglement element 4 lb of the first pair of entanglement elements Ia-Ib which transmits, to the quantum states resonator 8 (QSR), the quantum states information of the source medium 2, received from the first entanglement element 3 Ia of the first pair of entanglement elements Ia-Ib. The second, third and fourth entanglement element connected to the quantum states resonator 8 (QSR) are entanglement elements 7, 7a, 7b IIa, IIIa and IVa of the three second pairs of entanglement elements IIa-IIb, IIIa-IIIb, IVa-IVb that receive quantum states information from the quantum states resonator 8 (QSR) and transmit it to the corresponding second entanglement elements 9, 9a, 9b IIb, IIIb, IVb of the three second pairs of entanglement elements IIa-IIb, IIIa-IIIb, IVa-IVb. Each of the second entanglement elements 9, 9a, 9b IIb, IIIb, IVb of the three second pairs of entanglement elements IIa-IIb, IIIa-IIIb, IVa-IVb receiving information from the first entanglement elements 7, 7a, 7b IIa, IIIa and IVa of the three second pairs of entanglement elements IIa-IIb, IIIa-IIIb, IVa-IVb is connected to a separate end tank 10, 10a, 10b (ET1, ET2, ET3).

Example 6

In the system for transferring quantum states information according to the present invention, the effect of quantum information transmitted from the system for transferring quantum states information, according to a first embodiment, on the catalytic activity of an enzyme in an enolase reaction substrate solution was studied.

Enolase is an important enzyme involved in basic life processes, such as the combustion of glucose for energy production during the glycolysis process. It is a conservative protein that was evolutionarily shaped at the beginning of life on Earth. Apart from its catalytic function, enolase also plays other roles in metabolism in human cells and tissues and lower organisms; it is a multifunctional protein. Acting on the surface of cells as a receptor of plasminogen, it is converted into plasmin, which in turn digests tissue proteins. It is a mechanism used in embryogenesis (normal tissue growth), wound reconstruction, infection and settlement of the host organism by bacteria, fungi, and parasites. Microorganisms have their own enolase used for tissue infection. This mechanism is also important in the growth of cancerous tumors. In addition, enolase of microscopic fungi is a factor responsible for some allergies.

The effect of interactions generated by the two second entanglement elements 9, 9a of the second and third pair of entanglement elements on the course of the catalytic reaction of human muscle enolase was studied.

The second entanglement elements 9, 9a of the second and third pair of entanglement elements were aluminium plates, 1 cm by 2 cm in size, which were kept for 30 min at room temperature prior to testing. Enolase was isolated from human muscle tissue - by own method (Bednarz-Misa I, Pietkiewicz J, et al., 2009. Adv.Clin.Exp.Med. 18 (1),71-78). The enzyme stock solution was a preparation of 7.5 mM imidazole-HCl, pH 6.8, in stabilizing buffer containing 2.5 mM of $MgSO_4$, 50 mM of NaCl, and 50% of glycerol (stored at 4°C). The concentration of enolase in the stock solution $R_0$ was 5 mg/ml; while the catalytic activity was 52 IU/mg. The enzyme working solution ($R_0$ diluted 10 times with test buffer of pH 6.8) had a concentration of 1 mg/ml.

A standard test buffer was used in tests: 50 of mM imidazole-HCl, pH 6.8, 3 mM of $MgSO_4$, 0.4M of KCl and 2-PGA substrate (sodium salt), dissolved in $H_2O$ - stock solution $R_0$ at a concentration of 63 mM. 2-PGA in the enzyme assay was used at a standard concentration of 1 mM.

Protein concentration was determined spectrophotometrically by measuring the absorbance of enolase solution at 280 nm wavelength in quartz cells. In the present system, quartz cuvettes constituted the end tanks 10 made of polar dielectric 5. Enolase activity was determined by monitoring using a Jasco UV-Vis V-530 spectrophotometer. Increase of absorbance of the test solution at 240 nm wavelength (a measure of the reaction product production, PEP) within 60 seconds. The specific activity of the enzyme was expressed in international units relative to 1 mg of protein (Bednarz-Misa I, Pietkiewicz J, et al., 2009. Adv.Clin.Exp.Med. 18(1);71-78).

Experimental conditions: changes in enolase activity in a standard test system containing, in each case, 0.75 ml of test buffer, pH 6.8, and 6 $\mu$l of enolase working solution (6 $\mu$g of enzyme) in a quartz cuvette were tested over a period of 0-5 hours. The enzymatic reaction was initiated by adding 12 $\mu$l of 2-PGA substrate taken at 1-hour intervals from the substantive test or control test described in a) and b) below.

Substrate incubation:

a) Substantive test: a portion of stock solution Ro 2-PGA (0.120 ml) was placed in a quartz cuvette and treated for 5 hours with two second entanglement elements 9, 9a of the second and third pair of entanglement elements (which were adjacent to both sides of the cuvette) at room temperature.

b) Control test: 0.120 ml of stock solution Ro 2-PGA was placed in a quartz cuvette and incubated as above, but without contact with second entanglement elements 9, 9a of the second and third pair of entanglement elements (3 m distance from the substantive test).

Results are shown in Table 1.

Table 1 - Analytical activity of the enzyme-enolase

| Incubation time [h] | | 0 | 1 | 2 | 3 | 4 | 5 |
|---|---|---|---|---|---|---|---|
| Substantive test | Activity [IU/mg] | 52.4 | 53.1 | 50.45 | 52.27 | 48.1 | 47.08 |
| | $W_i / W_0$ [%] | 100 | 101.74 | 96.3 | 99.75 | 92.0 | 89.8 |
| Control test | Activity [IU/mg] | 52.4 | 45.13 | 40.06 | 36.44 | 28.24 | 25.13 |
| | $K_i / K_0$ [%] | 100 | 86.1 | 76.5 | 69.54 | 53.9 | 47.95 |
| $W_i / K_i$ [%] | | 100 | 117.7 | 125.9 | 143.4 | 170.3 | 187.3 |

A gradual loss of enolase activity during enzyme exposure at room temperature is a typical phenomenon and therefore, it was observed in control tests with added substrate unmodified by second entanglement elements 9, 9a of the second and third pairs of entanglement elements: after 1 hour, the enzyme lost specific activity by approx. 14%, and after 5 hours, the loss was as much as 52% of the initial activity.

As a result of the interaction of second entanglement elements 9, 9a of the second and third pair of entanglement elements on the 2-PGA substrate, the catalytic efficiency of human enolase increased significantly. As the contact time of second entanglement elements 9, 9a of the second and third pair of entanglement elements with the substrate was prolonged, an increase in enzyme-specific activity was observed - after 5 hours of the experiment, it was over 87% of the initial value.

The quantum information transmitted from the source medium 2, in the present embodiment a silicon monocrystal of very low entropy value, to the intermediate medium 6 placed in the quantum states resonator 8, and then to the end tank 10, which is a quartz reaction cell, very positively affected the catalytic activity of the enzyme. This indicates that substrate molecules are ordered. Such a phenomenon may reduce the energy expenditures needed to obtain the correct orientation of 2-PGA functional groups (carboxylic and phosphate) in relation to the centre of the catalytic enzyme. In addition, it is possible for such ordered substrate molecules to induce certain changes in the spatial structure of the catalytic pocket itself, facilitating the occurrence of the 2-PGA → PEP reaction.

Example 7

In the system for transferring quantum states information according to the present invention, the effect of quantum information transmitted from the system for transferring quantum states information, according to a first embodiment, on the catalytic activity of an enzyme in an enolase reaction substrate solution was studied. The experiment was conducted in a similar manner to the experiment shown in example 6.

The effect of interactions generated by three second entanglement elements 9, 9', 9" of the second pair of entanglement elements of the three (different) systems for transferring quantum states information of the present invention on the course of the catalytic reaction of human muscle enolase was studied.

Second entanglement elements 9, 9', 9" of the second pair of entanglement elements were aluminium plates (1 cm by 2 cm), which were maintained at room temperature for 30 min prior to testing. In the present embodiment of the invention, test samples are determined as follows:

　　Substantive test A - second entanglement element 9 of the second pair of entanglement elements of the first system for transferring quantum states information, wherein the quantum states generator 1 is a silicon monocrystal weighing approx. 100 g.
　　Substantive test B - second entanglement element 9' of the second entanglement element pair of the second system for transferring quantum states information, wherein the quantum states generator 1' is a silicon monocrystal weighing approx. 2 kg.

Blank test C - a plate made of aluminium (without quantum entanglement) not being a part of the system for transferring quantum states information of the present invention.

Substantive test D - a second entanglement element 9" of a second pair of entanglement elements of the third system for transferring quantum states information, wherein the quantum states generator 1" is a silicon monocrystal weighing approx. 500 g. Unlike the first and second system for transferring quantum states information (substantive test A and B, respectively), the third system for transferring quantum states information contained only one second pair of entanglement elements IIa-IIb, comprising a first entanglement element 7" of the second entanglement element pair and a second entanglement element 9" of the second entanglement element pair, respectively. In the first and second system for transferring quantum states information (substantive test A and B, respectively), there are many second pairs of

entanglement elements IIa-IIb, wherein the second entanglement elements 9, 9', one of each from of the second pair of entanglement elements is used in the present embodiment (substantive test A and B).

Control - laboratory environment without contact with an aluminium plate.

Enolase was isolated from human muscle tissue - by own method (Bednarz-Misa I, Pietkiewicz J, et al., 2009. Adv.Clin.Exp.Med. 18 (1),71-78). The enzyme stock solution was a preparation in the stabilizing buffer comprising 7.5 mM of imidazole-HCl pH 6.8, containing 2.5 mM of $MgSO_4$, 50 mM of NaCl, 50% of glycerol (stored at 4°C). The concentration of enolase in the stock solution $R_0$ was 5 mg/ml; while the catalytic activity was 52 IU/mg. The enzyme working solution (Ro diluted 10 times with test buffer of pH 6.8) had a concentration of 1 mg/ml.

A standard test buffer was used in tests: 50 of mM imidazole-HCl, pH 6.8, 3 mM of $MgSO_4$, 0.4M of KCl and 2-PGA substrate (sodium salt), dissolved in $H_2O$ - stock solution Ro at a concentration of 63 mM. The 2-PGA substrate was prepared in portions of 200 $\mu$l in five glass cuvettes (comprising end tanks 10 made of polar dielectric 5) and incubated over a period of 5 hours in the presence (in direct contact) of second entanglement elements 9, 9', 9" of a second pair of entanglement elements of three (different) systems for transferring quantum states information of the present invention, in the presence of an aluminium plate (without quantum entanglement - blank test C) and without contact with the plates (control).

A portion of 17 $\mu$l test substrate containing 0.773 ml of test buffer (buffer without contact with platelets) and 8 $\mu$l of enolase (Eno working solution in 1 mg/ml of test buffer without contact with platelets) was taken at one-hour intervals. The reaction was initiated by adding a sample of 17 $\mu$l of 2-PGA substrate to test buffer and enolase. 2-PGA in the enzyme assay was used at a standard concentration of 1 mM.

Protein concentration was determined spectrophotometrically by measuring the absorbance of enolase solution at 280 nm wavelength in quartz cells. In the present system, quartz cuvettes constituted the end tanks 10 made of polar dielectric 5. Enolase activity was determined by monitoring using a Jasco UV-Vis V-530 spectrophotometer. Increase of absorbance of the test solution at 240 nm wavelength (a measure of the reaction product production, PEP) within 60 seconds. The specific activity of the enzyme was expressed in international units relative to 1 mg of protein (Bednarz-Misa I, Pietkiewicz J, et al., 2009. Adv.Clin.Exp.Med. 18(1);71-78).

Experimental conditions: Enolase activity was tested in 0.773 ml of test buffer (buffer without contact with platelets) and 8 $\mu$l of enzyme (enolase working solution in test buffer, 1 mg/ml without contact with platelets). The enzymatic reaction was initiated by adding 17 $\mu$l of 2-PGA substrate taken at 1-hour intervals from a 2-PGA sample (incubated at room temperature in the presence of second entanglement elements 9, 9', 9" of the second pair of entanglement elements of three (different) systems for transferring quantum states information according to the present invention) or taken from the control test containing a substrate not treated with platelets (distance between incubated tests - approx. 3 m). Results are shown in Table 2.

Table 2 - Analytical activity of the enzyme-enolase

| Incubation time [h] | | 0 | 1 | 2 | 3 | 4 | 5 |
|---|---|---|---|---|---|---|---|
| Substantive test A | Activity [IU/mg] | 41.75 | 43.43 | 42.56 | 41.65 | 41.7 | 41.59 |
| | $W_i / W_0$ [%] | 100 | 104 | 102 | 99.8 | 99.9 | 99.6 |
| | $W_i / K_{0i}$ [%] | 105.9 | 109.8 | 108.3 | 103.3 | 107.4 | 113.5 |
| Substantive test B | Activity [IU/mg] | 45.85 | 47.43 | 46.2 | 46.5 | 46.95 | 46.08 |
| | $W_i / W_0$ [%] | 100 | 103.4 | 100.8 | 101.4 | 102.4 | 102.5 |
| | $W_i / K_{0i}$ [%] | 114.8 | 119.8 | 117.6 | 115.3 | 122.9 | 125.7 |
| Blind trial C | Activity [IU/mg] | 38.49 | 38.4 | 37.75 | 36.2 | 35.1 | 35.33 |
| | $W_i / W_0$ [%] | 100 | 99.8 | 98.1 | 94.1 | 91.2 | 91.8 |
| | $W_i / K_{0i}$ [%] | 97.6 | 97.1 | 96.1 | 89.8 | 90.4 | 96.4 |
| Substantive test D | Activity [IU/mg] | 49.91 | 47.55 | 44.79 | 44.36 | 44.1 | 43.93 |
| | $W_i / W_0$ [%] | 100 | 95.3 | 89.7 | 88.8 | 88.4 | 88.0 |
| | $W_i / K_{0i}$ [%] | 126.6 | 120.2 | 114 | 110 | 113.6 | 119.6 |

(continued)

| Incubation time [h] | | 0 | 1 | 2 | 3 | 4 | 5 |
|---|---|---|---|---|---|---|---|
| Control | Activity [IU/mg] | 39.43 | 39.56 | 39.3 | 40.33 | 38.82 | 36.65 |
| | $K_{0i} / K_0$ [%] | 100 | 100.3 | 99.7 | 109.8 | 98.5 | 93 |
| | $K_{0i} / K_{0i}$ [%] | 100 | 100 | 100 | 100 | 100 | 100 |

As shown in Table 2, slight enolase loss during enzyme exposure to room temperature, observed in a control test K with added substrate unmodified by second entanglement elements 9, 9', 9" of the second pair of entanglement elements, indicated the stability of enzyme protein maintained at room temperature for 5 hours.

The quantum information (of entropy close to 0), transmitted by second entanglement elements 9, 9', 9" of the second entanglement element pair from the quantum states generator 1, 1', 1" to the 2-PGA substrate in aqueous solution, positively affected the catalytic activity of the enzyme. As the contact time of second entanglement elements 9, 9', 9" of the second pair of entanglement elements with the end tank 10, 10', 10" (in the form of quartz cuvettes) and the substrate therein is prolonged, an increase in enzyme-specific activity can be observed. After 5 hours of the experiment, an increase of the test activity relative to the control K ranged from over a dozen % to more than 20% (see Table 2) can be observed.

In addition, a slight decrease in the activity of the enzyme in blank test C, relative to control, can be observed. In this case, the reduction of activity is attributed to the adverse effect of aluminium itself (of which the metal plate in contact with the cuvette is made) on the enzyme in the cuvette.

The system for transferring quantum states information according to the present invention, by transmitting information about low entropy from a quantum states generator 1, 1', 1", enables the reduction of energy expenditures needed to obtain the correct orientation of 2-PGA functional groups (carboxylic and phosphate) in relation to the catalytic center of the enzyme. Substrate molecules, ordered by interacting with second entanglement elements 9, 9', 9" of the second pair of entanglement elements of the three (different) systems for transferring quantum states information of the present invention, are believed to generate changes in the spatial structure of the catalytic pocket itself, facilitating the occurrence of the 2-PGA → PEP reaction.

Example 8

In the system for transferring quantum states information according to the present invention, the effect of quantum information transmitted from the system for transferring quantum states information according to a first embodiment on the enolase glycation process with methylsiloxane was studied.

Glycation is a non-enzymatic process, in which carbonyl groups present in reducing sugars or $\alpha$-oxo-aldehydes react with amino groups of peptides and proteins, nucleic acids, phospholipids (Maillard reaction). As a result of covalent bonds formation (Schiff bases) and a series of rearrangements, condensation and polymerization, a mixture of highly cross-linked products adversely affecting metabolic processes is formed. In physiological conditions, the process takes place slowly, but intensifies in states of chronic hyperglycaemia, impairs protein functions and is accompanied by carbonyl and oxidative stress, especially in complications of diabetes. The resulting high molecular advanced glycation (AGE) products exhibit significant resistance to enzymatic degradation. AGE accumulation can occur intracellularly, as well as in extracellular structures, including skin, nerve tissue, blood vessels, kidneys. Enolase is an important enzyme involved in basic life processes, such as the combustion of glucose for energy production during the glycolysis process. It is a conservative protein that was evolutionarily shaped at the beginning of life on Earth. Apart from its catalytic function, enolase also plays other roles in metabolism in human cells and tissues and lower organisms; it is a multifunctional protein. When it appears on the surface of cells as a receptor of plasminogen, it converts it into plasmin, which in turn digests tissue proteins. It is a mechanism used in embryogenesis (normal tissue growth), wound reconstruction, infection and settlement of the host organism by bacteria, fungi, and parasites. Microorganisms have their own enolase used for tissue infection. This mechanism is also important in the growth of cancerous tumors. In addition, enolase of microscopic fungi is a factor responsible for some allergies. Human muscle enolase has previously been shown to be glycated by several glycosylating agents, while phosphatidylserine significantly protects the enzyme against a decrease in enolase activity in the presence of AGE [Protein J. 2011, 30(3): 149-158]. This is important because enolase glycation can disrupt many processes in the body.

Methylglyoxal was chosen as an enolase modifying agent because it is formed in the body mainly as a product of defective glycolysis or in the lipid peroxidation process, as well as a result of disorders of defence systems against carbonyl and oxidative stress. Simultaneously, enolase glycation was performed without exposure to the second entanglement elements 9, 9a of the second and third pair of entanglement elements. The second control was a sample of enolase that was not glycated and not treated with the second entanglement elements 9, 9a of the second and third pair of entanglement elements.

The second entanglement elements 9, 9a of the second and third pair of entanglement elements were aluminium plates, 1 cm by 2 cm in size, which were kept for 30 min at room temperature prior to testing. Enolase was isolated from human muscle tissue using own method (Bednarz-Misa I, Pietkiewicz J, et al., 2009. Adv.Clin.Exp.Med. Vol.18 no.1; p.71-78). The enzyme stock solution was a preparation of 7.5 mM of imidazole-HCl, pH 6.8, in stabilizing buffer, containing 2.5 mM of $MgSO_4$, 50 mM of NaCl, 50% of glycerol (formulation stored at 4°C). The concentration of enolase in the stock solution Ro was 8 mg/ml (87 $\mu$M), while the enzyme specific activity was 25 IU/mg. In glycation mixtures, the enzyme concentration was 1 mg/ml (Ro diluted 8x with PBS buffer, pH 7.4).

In the experiment, a standard test buffer of 50 mM imidazole-HCl pH 6.8.3 mM $MgSO_4$, 0.4M KCl and a substrate of 2-PGA (sodium salt) dissolved in $H_2O$ - a stock solution $R_0$ at a concentration of 58.5 mM was used. 2-PGA in the enzyme assay was used at a standard concentration of 1 mM.

Protein concentration was determined spectrophotometrically by measuring the absorbance of enolase solution at 280 nm wavelength using quartz cuvettes. In the present system, quartz cuvettes constituted the end tanks 10 made of polar dielectric 5. Enolase activity was determined by monitoring in a Jasco UV-Vis V-530 spectrophotometer within 60 seconds, the increase in absorbance of the test solution at 240 nm wavelength (a measure of reaction product production, PEP). The specific activity of the enzyme was expressed in international units relative to 1 mg of protein (Bednarz-Misa I, Pietkiewicz J, et al., 2009. Adv.Clin.Exp.Med. 18(1);71-78).

Two glycation systems were prepared using a large excess of methylglyoxal in relation to enolase (ratio 1:300 mol:mol) - in accordance with the conditions according to [Pietkiewicz J. et al., 2011, Protein J. 30 (3):149-158]:

a) Substantive test: glycation of enolase treated with second entanglement elements 9, 9a of the second and third pair of entanglement elements.

175 $\mu$l of PBS pH 7.4 buffer were placed in the quartz cuvette, 20 $\mu$l of enolase stock solution and 5.5 $\mu$l of 0.8% aqueous methylglyoxal solution were added. In such a system, the effect of quantum information transmitted from source medium 2 (silicon monocrystal) on the modification of the glycolysis/gluconeogenesis enzyme enolase by methylglyoxal as a reactive glycating agent was studied.

In order to transmit quantum information from the source medium 2, the mixture was subjected for 3 hours to two second entanglement elements 9, 9a of the second and third pair of entanglement elements (which adhered to both sides of the cuvette). The system was kept at room temperature.

b) Control test 1: glycation of enolase not treated with second entanglement elements 9, 9a of the second and third pairs of entanglement elements.

The reaction system was prepared as a substantive test (a). Incubation at room temperature occurred at the same time, without contact with the second entanglement elements 9, 9a of the second and third pair of entanglement elements (3 m distance from the substantive test).

c) Control test 2: Unmodified enolase, not treated with second entanglement elements 9, 9a of the second and third pair of entanglement elements.

The control system (2) was prepared by adding to 180 $\mu$l of PBS buffer, pH 7.4, 20 $\mu$l of enolase only; incubation at room temperature was carried out at the same time without contact with the second entanglement elements 9.9a of the second and third pair of entanglement elements (3 m distance from the substantive test).

Changes in enolase activity in the standard test system containing in each case 0.870 ml of pH 6.8 test buffer and 15 $\mu$l of 2-PGA substrate stock solution were tested over 3 hours of modification. The enzymatic reaction was initiated by each addition of an enzyme of 15 $\mu$l (12 $\mu$g protein) taken from incubation mixtures (a), (b) or (c) at 1-hour intervals.

The glycation process was interrupted after 3 hours by adding an aqueous solution of lysine at a concentration of 4 mg/ml to methylglyoxal samples (excess lysine bound unreacted methylglyoxal).

In order to detect the presence of high molecular weight products of advanced glycation of enolase (AGE), samples of each mixture were electrophoresized. Electrophoresis conditions according to [Pietkiewicz J. et al., 2011, Protein J. 30 (3):149-158]. 12 ug of protein from the analyzed samples was applied to the gel paths.

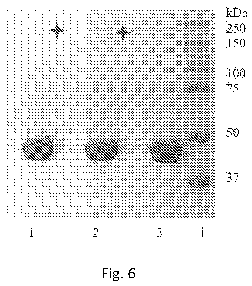

Results are shown in fig. 6 and Table 3.

Table 3 - Analytical activity of the enzyme-enolase

| Incubation time [h] | | 0 | 1 | 2 | 3 |
|---|---|---|---|---|---|
| Substantive test (W) (enolase + methylglyoxal + entanglement elements) | Activity [IU/mg] | 29.22 | 25.78 | 24.12 | 23.4 |
| | $W_i / W_0$ [%] | 100 | 88.2 | 82.5 | 80.1 |
| | $W_i / K_{(2)i}$ [%] | 118.3 | 113.2 | 117.3 | 120.1 |
| Control test 1(K1) (enolase + methylglyoxal, without entanglement elements) | Activity [IU/mg] | 24.6 | 21.37 | 19.15 | 17.64 |
| | $K_{(1)i} / K_{(1)0}$ [%] | 100 | 86.9 | 77.85 | 71.7 |
| | $K_{(1)i} / K_{(2)i}$ [%] | 99.6 | 93.8 | 88.8 | 90.6 |
| Control test 2(K2) (enolase without methylglyoxal, without entanglement elements) | Activity [IU/mg] | 24.7 | 22.78 | 20.56 | 19.48 |
| | $K_{(2)i} / K_{(2)0}$ [%] | 100 | 92.2 | 83.2 | 78.9 |

As a result of exposure to the enzyme at room temperature, there was partial loss of enolase activity - in a control test (K2) containing a native enzyme not treated with second entanglement elements 9, 9a of the second and third pair of entanglement elements, specific activity after 3 hours of incubation decreased from 24.7 IU/mg to 19.48 IU/mg and was over 21% lower than baseline (Table 3). Modification of enolase with methylsiloxane deactivated the enzyme to a greater extent - after 3 hours of glycation in sample K1, a decrease in specific activity was observed from 24.6 IU/mg to 17.64 IU/mg, i.e., by approx. 28%.

In the presence of second entanglement elements 9, 9a of the second and third pair of entanglement elements, the time dynamics of changes in specific activity were similar to those observed in the control test K2, but higher specific activity values were observed: from 29.22 IU/mg initially to 23.4 IU/mg in the third hour of the experiment.

When comparing the relative changes in the specific activity of the enzyme in the substantive test (S) relative to the values obtained for enolase in the unmodified control test (K2), a beneficial effect of the quantum information transmitted from the source medium 2, which in the present embodiment was a silicon monocrystal with a very low entropy value, to the intermediate medium 6 placed in the quantum states resonator 8, and then to the end tank 10, which is a quartz reaction cell, on the catalytic efficiency of the enzyme, was clearly observed. Throughout the experiment, the glycated enzyme maintained high activity in the presence of second entanglement elements 9, 9a of the second and third pair of entanglement elements - it accounted for 113%-120% of the activity of a specific, unmodified control test (Table 2).

At the same time, contact with second entanglement elements 9, 9a of the second and third pair of entanglement elements had no effect on the formation of AGE during glycation of enolase by methylsiloxane. The profile of the produced high-molecular products of advanced glycation is shown in fig. 6. AGE of Mw 180 and Mw 230 kDa (marked with stars) was observed both in the substantive test treated with second entanglement elements 9, 9a of the second and third pair of entanglement elements (path 1) and in the control test, in which glycation occurred in the absence of second entanglement elements 9, 9a of the second and third pair of entanglement elements (path 2). For comparison, the protein profile of the native non-glycated enolase sample is shown in path 3.

Glycation that accompanies humans throughout their lives is an adverse phenomenon, as it reduces metabolic efficiency of the body. For example, human muscle enolase modified by methylglyoxal shows a decrease in the catalytic activity of the enzyme accompanying the formation of high-molecular glycation products.

The second entanglement element 9, 9a of the second and third pair of entanglement elements that transmit quantum states information from the source medium 2, which is a silicon monocrystal, do not inhibit the production of AGE, but have a very beneficial effect on maintaining the high catalytic capacity of the enzyme.

This indicates the possibility of a positive protective effect of this phenomenon in the system. It could ensure the maintenance of an efficient and energy-efficient glycolysis process, despite conditions facilitating glycation in cells (hyperglycaemia, carbonyl stress, oxidative stress).

Example 9

In the system for transferring quantum states information according to the present invention, the effect of quantum information transmitted from the system for transferring quantum states information, according to a first embodiment, on the forced ignition parameters of brown coal dust under closed spherical reactor conditions was studied.

The tests were performed for coal in its basic state and then for brown coal samples after interaction with the second entanglement element 9 of the second pair of entanglement elements.

The measurements were carried out on brown coal samples from the Turoszów deposit, the coal samples used for the tests were stored in a closed volume (approx. 6 years). Grain distribution of coal was typical for power boilers used in furnaces, i.e., sizes were below 200 $\mu$m. Due to the storage time of coal, the content of flammable volatile parts has decreased. The ash content in coal was approx. 20% relative to dry matter.

The tests were carried out for three different dust concentrations in the air (0.4 kg/m$^3$, 0.8 kg/m$^3$ and 1 kg/m$^3$).

As a result of the research, the following was determined:

Maximum pressure in reactor $P_{max}$, bar, i.e., the maximum pressure resulting from the increase in the volume of combustion gases. The value of this pressure corresponds to the amount of emitted exhaust gases and heat, while the second parameter measured simultaneously, i.e., the pressure rise rate P'max, bar/s, i.e., the maximum pressure increase, gives information about the rate of exhaust emissions (this corresponds to the combustion speed) and the intensity of the combustion process.

The lower limit of the occurrence of forced ignition (DGW, g/m$^3$) determines the lowest concentration of fuel (in this case coal dust with air in the measuring sphere) at which ignition and combustion occurred. Below this value, the combustible mixture is too low in the combustible component and contains too much oxidant to initiate ignition.

To sum up, the measured parameters show the amount of energy obtained in the combustion process, the amount of exhaust gases and the increase in temperature over time.

Results of assays are shown in Table 4.

Table 4 - parameters of brown coal combustion

|  | Concentration [kg/m$^3$] | $P_{max}$ [bar] | $P'_{max}$ [bar/s] |
|---|---|---|---|
| Raw coal | 0.4018 | 0.9172 | 8.64 |
|  | 0.8036 | 2.7020 | 7.68 |
|  | 1.0000 | 3.5461 | 10.63 |
| Modified coal | 0.4018 | 2.6187 | 8.08 |
|  | 0.8036 | 4.2905 | 20.86 |
|  | 1.0000 | 5.2428 | 26.70 |

The comparison of results obtained for "raw" brown coal and for modified coal (i.e. after interaction with the second entanglement element 9 of the second pair of entanglement elements) for all three concentrations of brown coal dust in the mixture with air show significant differences in favour of modified coal, i.e. a significant, several-fold increase in both the tested maximum pressure parameters and the pressure increase rate, which indicates an improvement in the reactivity of modified coal compared to the initial one, including an increase in the burning rate under closed spherical reactor conditions. These results may indicate a change in the chemical structure of coal.

Evaluation of parameter change in the completed research

Maximum chamber pressure for three concentrations of coal dust:

- 0.4 kg/m$^3$ is: 288%

- 0.8 kg/m$^3$ is: 63%

- 1 kg/m$^3$ is: 147%

In turn, for the pressure increase rate (P'$_{max}$), the results are as follows:
Respectively for concentrations:

- 0.4 kg/m$^3$ - for this dust concentration, the value is comparable with the average results for unmodified coal dust.

Increase in the parameter of pressure increase rate (P'$_{max}$) for dust concentration:

- 0.8 kg/m$^3$ is: 272%

- 1 kg/m$^3$ is: 251%.

The lower explosion limit is also shifted towards lower values, from about 0.40 kg/m$^3$ for raw coal to 0.30 kg/m$^3$ for modified coal. The obtained results suggest a significant increase in flammability properties by significantly reducing the initiation energy of combustion.

Example 10

In the system for transferring quantum states information according to the present invention, the effect of quantum information transmitted from the system for transferring quantum states information, according to a first embodiment, on lysozyme activity was studied.

The activity of lysozyme was determined using an indirect method by measuring the dynamics of turbidity change of *Micrococcus lysodeicticus* cell wall suspension in phosphate buffer at pH=6.3 after addition of lysozyme-containing solutions (protein concentration of 1 mg/cm$^3$). The measurement was carried out at a constant temperature of 25°C using a turbidimetric method at wavelength $\lambda$ = 450nm, determining the drop in absorbance every 60 seconds over 6 minutes.

$$Lyzozyme\ activity = \frac{A_0 - A_6}{6} \cdot 10000 \left[\frac{units}{mg\ of\ protein}\right]$$

wherein:

$A_0$ - test absorbance without lysozyme
$A_6$ - test absorbance after incubating for 6 minutes

The degree of change in the activity of the lysozyme disposed in the end tank 10 to which the second entanglement element 9 of the second entanglement element pair of the system for transferring quantum states information of the present invention is attached is determined by measuring the specific activity (enzyme concentration of 1 mg/ml) at 1h intervals. In the sample placed in the end tank 10, the maximum increase in specific activity by 27.65%, compared to the control test, was observed on the 5th hour of the process. On day 3, the lysozyme solution in the end tank 10 exhibited 5.63% higher activity compared to the control test.

List of References

1 - quantum states generator (QSG)

2 - source medium of quantum information

3 - first entanglement element Ia of the first pair of entanglement elements Ia-Ib

4 - second entanglement element Ib of the first pair of entanglement elements Ia-Ib

5 - polar dielectric

6 - intermediate medium

7 - first entanglement element IIa of the second pair of entanglement elements IIa-IIb

7a - first entanglement element IIIa of the third pair of entanglement elements IIIa-IIIb

7b - first entanglement element IVa of the fourth pair of entanglement elements IVa-IVb

8 - quantum states resonator (QSR)

9 - second entanglement element IIb of the second pair of entanglement elements IIa-IIb

9a - second entanglement element IIIb of the third pair of entanglement elements IIIa-IIIb

9b - second entanglement element IVb of the fourth pair of entanglement elements IVa-IVb

10 - end tank (ET/ET1)

10a - second end tank (ET2)

10b - third end tank (ET3)

11 - end medium

12 - insulation

**Claims**

1. A system for transferring quantum states information, particularly of macromolecular systems, comprising a source medium (2) of quantum information, a transmitter and receiver of quantum states information and an end medium (11), and a quantum states resonator (8) in contact with an intermediate medium (6), and **characterised in that** the transmitter and receiver of quantum states information being at least two pairs of entanglement elements (3, 4, 7, 9) Ia-Ib, IIa-IIb, wherein the first entanglement element (3) Ia of the first pair of entanglement elements Ia-Ib is connected to a quantum states generator (1) in contact with the source medium (2) of quantum information, and the second entanglement element (4) Ib of the first pair of entanglement elements Ia-Ib is connected to the quantum states resonator (8), and comprises at least one end tank (10) in contact with the end medium (11), wherein the first entanglement element (7) IIa of at least one second pair of entanglement elements IIa-IIb is connected to a quantum states resonator (8), and the second entanglement element (9) IIb of at least one second pair of entanglement elements IIa-IIb is connected to at least one end tank (10).

2. The system for transferring quantum states information according to claim 1, wherein the individual pairs of entanglement elements (3, 4, 7, 9) - first Ia-Ib and at least one second IIa-IIb - are quantum entangled, so that the first entanglement element (3) Ia of the first pair of entanglement elements Ia-Ib is quantum entangled with the second entanglement element (4) Ib of the first pair of entanglement elements Ia-Ib, and the first entanglement element (7) IIa of at least one second pair of entanglement elements IIa-IIb is quantum entangled with the second entanglement element (9) IIb of at least one second pair of entanglement elements IIa-IIb, wherein the quantum entanglement of the first pair of entanglement elements Ia-Ib is independent of the quantum entanglement of at least one second pair of entanglement elements IIa-IIb.

3. The system for transferring quantum states information according to claim 1 or 2, wherein the individual pairs of entanglement elements (3, 4, 7, 9) Ia-Ib, IIa-IIb are made of a material that is a chemical element in the boron group of the periodic table or an element, which has an odd number of electrons on the valence shell of an atom and is in the form of a solid, or a material in which there are covalent bonds between atoms, and which is in the form of a solid.

4. The system for transferring quantum states information according to any of claims 1 to 3, wherein the source medium (2) of quantum information is a solid, liquid, gas, plasma or any mixture thereof and/or constitutes a monocrystal formed from a chemical element in the carbon group of the periodic table.

5. The system for transferring quantum states information according to any of claims 1 to 4, wherein the intermediate medium (6) and/or the end medium (11) is a gas, liquid, solid or a mixture thereof.

6. The system for transferring quantum states information according to any of claims 1 to 5, wherein the quantum states resonator (8) and/or at least one end tank (10) is made of polar dielectric (5).

7. The system for transferring quantum states information according to any of claims 1 to 6, wherein the quantum states resonator (8) and/or at least one end tank (10) is covered with insulation (12).

8. A method of transferring quantum states information, particularly of macromolecular systems, **characterized in** comprising steps of:

   a) receiving a quantum states information from the source medium (2) of quantum information through the first

entanglement element (3) Ia of the first pair of entanglement elements Ia-Ib connected to the quantum states generator (1) in contact with the source medium (2) of quantum information;

b) transmitting the quantum states information of the source medium (2) of the quantum information from the first entanglement element (3) Ia of the first pair of entanglement elements Ia-Ib to the second entanglement element (4) Ib of the first pair of entanglement elements Ia-Ib;

c) transmitting the quantum states information from the second entanglement element (4) Ib of the first pair of entanglement elements Ia-Ib to the quantum states resonator (8) connected to the second entanglement element (4) of the first pair of entanglement elements Ia-Ib, and then to the intermediate medium (6) in contact with the quantum states resonator (8);

d) receiving the quantum states information of the intermediate medium (6) through the first entanglement element (7) IIa of at least one second pair of entanglement elements IIa-IIb connected to the quantum states resonator (8);

e) transmitting the quantum states information of the intermediate medium (6) from the first entanglement element (7) IIa of at least one second pair of entanglement elements IIa-IIb to the second entanglement element (9) IIb of at least one second pair of entanglement elements IIa-IIb;

f) transmitting the quantum states information of the intermediate medium (6) from the second entanglement element (9) IIb of at least one second pair of entanglement elements IIa-IIb to the end medium (11) in contact with at least one end tank (10) connected to the second entanglement element (9) IIb of at least one second pair of entanglement elements IIa-IIb.

9. The method of transferring quantum states information according to claim 8, wherein the individual pairs of entanglement elements (3, 4, 7, 9) - first Ia-Ib and at least one second IIa-IIb - are quantum entangled, so that the first entanglement element (3) Ia of the first pair of entanglement elements Ia-Ib is quantum entangled with the second entanglement element (4) Ib of the first pair of entanglement elements Ia-Ib, and the first entanglement element (7) IIa of at least one second pair of entanglement elements IIa-IIb is quantum entangled with the second entanglement element (9) IIb of at least one second pair of entanglement elements IIa-IIb, wherein the quantum entanglement of the first pair of entanglement elements Ia-Ib is independent of the quantum entanglement of at least one second pair of entanglement elements IIa-IIb.

10. The method of transferring quantum states information according to claim 8 or 9, wherein the individual pairs of entanglement elements (3, 4, 7, 9) Ia-Ib, IIa-IIb are made of a material that is a chemical element in the boron group of the periodic table or an element, which has an odd number of electrons on the valence shell of an atom and is in the form of a solid, or a material in which there are covalent bonds between atoms, and which is in the form of a solid.

11. The method of transferring quantum states information according to any of claims 8 to 10, wherein the source medium (2) of quantum information is a solid, liquid, gas, plasma or any mixture thereof.

12. The method of transferring quantum states information according to claim 11, wherein the source medium (2) of quantum information is a monocrystal formed from a chemical element in the carbon group of the periodic table.

13. The method of transferring quantum states information according to any of claims 8 to 12, wherein the intermediate medium (6) and/or the end medium (11) is a gas, liquid, solid or a mixture thereof.

14. The method of transferring quantum states information according to any of claims 8 to 13, wherein the quantum states resonator (8) and/or at least one end tank (10) is made of polar dielectric (5).

15. The method of transferring quantum states information according to any of claims 8 to 14, wherein the quantum states resonator (8) and/or at least one end tank (10) is covered with insulation (12).

**Patentansprüche**

1. Das System zur Übertragung von Quantenzustandsinformationen, insbesondere von makromolekularen Systemen, umfassend einen Sender (2) von Quanteninformationen, einen Sender und Empfänger von Quantenzustandsinformationen und ein Endmedium (11), **und**

einen Quantenzustandsresonator (8)
in Kontakt mit einem Zwischenmedium (6), und **dadurch gekennzeichnet, dass** der Sender und Empfänger von Quantenzustandsinformationen mindestens zwei Paare von Verschränkungselementen (3, 4, 7, 9) Ia-IIb, IIa-IIb sind, wobei das erste Verschränkungselement (3) Ia des ersten Paares von Verschränkungselementen Ia- Ib mit einem Quantenzustandsgenerator (1) in Kontakt mit dem Quellmedium (2) der Quanteninformation verbunden ist, und das zweite Verschränkungselement (4) Ib des ersten Paares von Verschränkungselementen Ia-Ib mit dem Quantenzustandsresonator (8) verbunden ist und mindestens einen Endtank (10) in Kontakt mit dem Endmedium (11) umfasst, wobei das erste Verschränkungselement (7) IIa mindestens eines zweiten Paars von Verschränkungselementen IIa-IIb mit einem Quantenzustandsresonator (8) verbunden ist und das zweite Verschränkungselement (9) IIb mindestens eines zweiten Paars von Verschränkungselementen IIa-IIb mit mindestens einem Endtank (10) verbunden ist.

2. Das System zur Übertragung von Quantenzustandsinformationen gemäß Anspruch 1, **wobei** einzelne Paare von Verschränkungselementen (3, 4, 7, 9) - erstes Ia-Ib und mindestens ein zweites IIa-IIb - quantenverschränkt sind, so dass das erste Verschränkungselement (3) Ia des ersten Paares von Verschränkungselementen Ia-Ib mit dem zweiten Verschränkungselement (4) Ib des ersten Paares von Verschränkungselementen Ia-Ib umfasst, und das erste Verschränkungselement (7) IIa mindestens eines zweiten Paares von Verschränkungselementen IIa-IIb mit dem zweiten Verschränkungselement (9) IIb mindestens eines zweiten Paares von Verschränkungselementen IIa- IIb quantenverschränkt ist, wobei die Quantenverschränkung des ersten Paares von Verschränkungselementen Ia-Ib unabhängig von der Quantenverschränkung von mindestens einem zweiten Paar von Verschränkungselementen IIa-IIb ist.

3. Das System zur Übertragung von Quantenzustandsinformationen gemäß Anspruch 1 oder 2, **wobei** die einzelnen Paare von Verschränkungselementen (3, 4, 7, 9) Ia-Ib, IIa-IIb aus einem Material bestehen, das ein chemisches Element in der Borgruppe des Periodensystems oder ein Element ist, das eine ungerade Anzahl von Elektronen auf der Valenzschale eines Atoms umfasst und in Form eines Feststoffes vorliegt, oder einem Material, in dem kovalente Bindungen zwischen Atomen bestehen, und das in Form eines Feststoffes vorliegt.

4. Das System zur Übertragung von Quantenzustandsinformationen gemäß einem der Ansprüche 1 bis 3, **wobei** das Quellmedium (2) der Quanteninformation ein Feststoff, eine Flüssigkeit, ein Gas, ein Plasma oder eine Mischung davon ist und/oder einen Monokristall darstellt, der aus einem chemischen Element in der Kohlenstoffgruppe des Periodensystems gebildet ist.

5. Das System zur Übertragung von Quantenzustandsinformationen gemäß einem der Ansprüche 1 bis 4, **wobei** das Zwischenmedium (6) und/oder das Endmedium (11) ein Gas, eine Flüssigkeit, ein Feststoff oder eine Mischung davon ist.

6. Das System zur Übertragung von Quantenzustandsinformationen gemäß einem der Ansprüche 1 bis 5, **wobei** der Quantenzustandsresonator (8) und/oder mindestens ein Endtank (10) aus polarem Dielektrikum (5) besteht.

7. Das System zur Übertragung von Quantenzustandsinformationen gemäß einem der Ansprüche 1 bis 6, **wobei** der Quantenzustandsresonator (8) und/oder mindestens ein Endtank (10) mit einer Isolierung (12) bedeckt ist.

8. Ein Verfahren zur Übertragung von Quantenzustandsinformationen, insbesondere von makromolekularen Systemen, **dadurch gekennzeichnet, dass** sie die folgenden Schritte umfassen:

a) Empfangen einer Quantenzustandsinformation von dem Quellenmedium (2) der Quanteninformation durch das erste Verschränkungselement (3) Ia des ersten Paares von Verschränkungselementen Ia-Ib, das mit dem Quantenzustandsgenerator (1) in Kontakt mit dem Quellmedium (2) der Quanteninformation verbunden ist,
b) Übertragen der Quantenzustandsinformation des Quellmediums (2) der Quanteninformation von dem ersten Verschränkungselement (3) Ia des ersten Paares von Verschränkungselementen Ia-Ib zu dem zweiten Verschränkungselement (4) Ib des ersten Paares von Verschränkungselementen Ia-Ib;
c) Übertragen der Quantenzustandsinformation von dem zweiten Verschränkungselement (4) Ib des ersten Paares von Verschränkungselementen Ia- Ib an den Quantenzustandsresonator (8), der mit dem zweiten Verschränkungselement (4) des ersten Paares von Verschränkungselementen Ia-Ib verbunden ist, und dann an das Zwischenmedium (6) in Kontakt mit dem Quantenzustandsresonator (8);
d) Empfangen der Quantenzustandsinformation des Zwischenmediums (6) durch das erste Verschränkungselement (7) IIa von mindestens einem zweiten Paar von Verschränkungselementen IIa-IIb, das mit dem Quantenzustandsresonator (8) verbunden ist,
e) Übertragen der Quantenzustandsinformation des Zwischenmediums (6) von dem ersten Verschränkungselement (7) IIa von mindestens einem zweiten Paar von Verschränkungselementen IIa-IIb an das zweite Verschränkungselement (9) IIb von mindestens einem zweiten Paar von Verschränkungselementen IIa- IIb;

f) Übertragen der Quantenzustandsinformation des Zwischenmediums (6) von dem zweiten Verschränkungselement (9) IIb mindestens eines zweiten Paares von Verschränkungselementen IIa-IIb an das Endmedium (11) in Kontakt mit mindestens einem Endtank (10), der mit dem zweiten Verschränkungselement (9) IIb mindestens eines zweiten Paares von Verschränkungselementen IIa-IIb verbunden ist.

9. Das Verfahren zur Übertragung von Quantenzustandsinformationen gemäß Anspruch 8, **wobei** die einzelne Paare von Verschränkungselementen (3, 4, 7, 9) - erstes Ia-Ib und mindestens ein zweites IIa-IIb - quantenverschränkt sind, so dass das erste Verschränkungselement (3) Ia des ersten Paares von Verschränkungselementen Ia-Ib mit dem zweiten Verschränkungselement (4) Ib des ersten Paares von Verschränkungselementen Ia-Ib quantenverschränkt ist, und das erste Verschränkungselement (7) IIa mindestens eines zweiten Paares von Verschränkungselementen IIa-IIb mit dem zweiten Verschränkungselement (9) IIb mindestens eines zweiten Paares von Verschränkungselementen IIa- IIb quantenverschränkt ist, wobei die Quantenverschränkung des ersten Paares von Verschränkungselementen Ia-Ib unabhängig von der Quantenverschränkung von mindestens einem zweiten Paar von Verschränkungselementen IIa-IIb ist.

10. Das Verfahren zur Übertragung von Quantenzustandsinformationen nach Anspruch 8 oder 9, **wobei** die einzelnen Paare von Verschränkungselementen (3, 4, 7, 9) Ia-Ib, IIa-IIb aus einem Material bestehen, das ein chemisches Element in der Borgruppe des Periodensystems oder ein Element ist, das eine ungerade Anzahl von Elektronen auf der Valenzschale eines Atoms umfasst und in Form eines Feststoffes vorliegt, oder einem Material, in dem kovalente Bindungen zwischen Atomen bestehen, und das in Form eines Feststoffes vorliegt.

11. Das Verfahren zur Übertragung von Quantenzustandsinformationen gemäß einem der Ansprüche 8 bis 10, **wobei** das Quellenmedium (2) der Quanteninformationen ein Feststoff, eine Flüssigkeit, ein Gas, ein Plasma oder eine Mischung davon ist.

12. Das Verfahren zur Übertragung von Quantenzustandsinformationen gemäß Anspruch 11, **wobei** das Quellenmedium (2) der Quanteninformationen ein Monokristall ist, der aus einem chemischen Element in der Kohlenstoffgruppe des Periodensystems gebildet ist.

13. Das Verfahren zur Übertragung von Quantenzustandsinformationen gemäß einem der Ansprüche 8 bis 12, **wobei** das Zwischenmedium (6) und/oder das Endmedium (11) ein Gas, eine Flüssigkeit, ein Feststoff oder eine Mischung davon ist.

14. Das Verfahren zur Übertragung von Quantenzustandsinformationen gemäß einem der Ansprüche 8 bis 13, **wobei** der Quantenzustandsresonator (8) und/oder mindestens ein Endtank (10) aus polarem Dielektrikum (5) besteht.

15. Das Verfahren zur Übertragung von Quantenzustandsinformationen gemäß einem der Ansprüche 8 bis 14, **wobei** der Quantenzustandsresonator (8) und/oder mindestens ein Endtank (10) mit einer Isolierung (12) bedeckt ist.

## Revendications

1. Un système pour transmettre d'informations d'états quantiques, en particulier des systèmes macromoléculaires, comprenant un milieu primaire (2) d'information quantique, un émetteur et un récepteur d'information d'états quantiques et un milieu terminal (11), et un résonateur d'états quantiques (8) en contact avec un milieu intermédiaire (6) et **caractérisé en ce que** l'émetteur et le récepteur d'information d'états quantiques étant au moins deux paires d'éléments d'intrication (3, 4, 7, 9) Ia-Ib, IIa-IIb, dans lesquels le premier élément d'intrication (3) Ia de la première paire d'éléments d'intrication Ia-Ib est relié à un générateur d'états quantiques (1) en contact avec le milieu primaire (2) d'information quantique, et le second élément d'intrication (4) Ib de la première paire d'éléments d'intrication Ia-Ib est relié au résonateur d'états quantiques (8), et comprend au moins un réservoir terminal (10) en contact avec le milieu terminal (11), dans lequel le premier élément d'intrication (7) IIa d'au moins une seconde paire d'éléments d'intrication IIa-IIb est relié à un résonateur d'états quantiques (8), et le second élément d'intrication (9) IIb d'au moins une seconde paire d'éléments d'intrication IIa-IIb est relié à au moins un réservoir terminal (10).

2. Le système pour transmettre d'information d'états quantiques selon la revendication 1, dans lequel les paires individuelles d'éléments d'intrication (3,4,7,9) - la première Ia-Ib et au moins une seconde IIa-IIb - sont intriquées, de sorte que le premier élément d'intrication (3) Ia de la première paire d'éléments d'intrication Ia-Ib est intriqué avec le second élément d'intrication (4) Ib de la première paire d'éléments d'intrication Ia-Ib, et le premier élément d'intrication (7) IIa d'au moins une seconde paire d'éléments d'intrication IIa-IIb est intriqué avec le second élément d'intrication (9) IIb d'au moins une seconde paire d'éléments d'intrication IIa-IIb, dans lequel l'intrication quantique de la première paire d'éléments d'intrication Ia-Ib est indépendant de l'intrication quantique d'au moins une seconde paire d'éléments d'intrication IIa-IIb.

3. Le système pour transmettre d'information d'états quantiques selon la revendication 1 ou 2, dans lequel les paires individuelles d'éléments d'intrication (3, 4, 7, 9) Ia-Ib, IIa-IIb sont constituées d'un matériau qui est un élément chimique de la colonne de bore du tableau périodique ou d'un élément, qui a un nombre impair d'électrons sur la couche de valence d'un atome et qui est sous la forme d'un solide, ou d'un matériau dans lequel il existe des liaisons covalentes entre les atomes, et qui est sous la forme d'un solide.

4. Le système pour transmettre d'information d'états quantiques selon l'une quelconque des revendications 1 à 3, dans lequel le milieu primaire (2) d'informations quantiques est un solide, un liquide, un gaz, un plasma ou un quelconque mélange de ceux-ci et/ou constitue un monocristal formé à partir d'un élément chimique de la colonne de carbone du tableau périodique.

5. Le système pour transmettre d'information d'états quantiques selon l'une quelconque des revendications 1 à 4, dans lequel le milieu intermédiaire (6) et/ou le milieu terminal (11) est un gaz, un liquide, un solide ou un mélange de ceux-ci.

6. Le système pour transmettre d'information d'états quantiques selon l'une quelconque des revendications 1 à 5, dans lequel le résonateur d'états quantiques (8) et/ou au moins un réservoir terminal (10) est constitué d'un diélectrique polarisé (5).

7. Le système pour transmettre d'information d'états quantiques selon l'une quelconque des revendications 1 à 6, dans lequel le résonateur d'états quantiques (8) et/ou au moins un réservoir terminal (10) est recouvert d'un isolant (12).

8. Un procédé de transmission d'informations d'états quantiques, en particulier des systèmes macromoléculaires, **caractérisé en ce qu'**il comprend les étapes de:

   a) recevoir une information d'états quantiques du milieu primaire (2) d'information quantique à travers le premier élément d'intrication (3) Ia de la première paire d'éléments d'intrication Ia-Ib relié au générateur d'états quantiques (1) en contact avec le milieu primaire (2) d'information quantique ;
   b) transmettre l'information d'états quantiques du milieu primaire (2) d'information quantique du premier élément d'intrication (3) Ia de la première paire d'éléments d'intrication Ia-Ib au second élément d'intrication (4) Ib de la première paire d'éléments d'intrication Ia-Ib ;
   c) transmettre l'information d'états quantiques du deuxième élément d'intrication (4) Ib de la première paire d'éléments d'intrication Ia-Ib au résonateur d'états quantiques (8) relié au deuxième élément d'intrication (4) de la première paire d'éléments d'intrication Ia-Ib, puis au milieu intermédiaire (6) en contact avec le résonateur d'états quantiques (8) ;
   d) recevoir l'information d'états quantiques du milieu intermédiaire (6) à travers le premier élément d'intrication (7) IIA d'au moins une deuxième paire d'éléments d'intrication IIa-IIb reliés au résonateur d'états quantiques (8) ;
   e) transmettre l'information d'états quantiques du milieu intermédiaire (6) du premier élément d'intrication (7) IIA d'au moins une deuxième paire d'éléments d'intrication IIa-IIb au deuxième élément d'intrication (9) IIb d'au moins une deuxième paire d'éléments d'intrication IIa-IIb ;
   f) transmettre l'information d'états quantiques du milieu intermédiaire (6) du second élément d'intrication (9) IIb d'au moins une seconde paire d'éléments d'intrication IIa-IIb au milieu terminal (11) en contact avec au moins un réservoir terminal (10) relié au second élément d'intrication (9) IIb d'au moins une seconde paire d'éléments d'intrication IIa-IIb.

9. Le procédé de transmission d'information d'états quantiques selon la revendication 8, dans lequel les paires individuelles d'éléments d'intrication (3, 4, 7, 9) - la première Ia-Ib et au moins une deuxième IIa-IIb - sont intriquées, de sorte que le premier élément d'intrication (3) Ia de la première paire d'éléments d'intrication Ia-Ib est intriqué avec le deuxième élément d'intrication (4) Ib de la première paire d'éléments d'intrication Ia-Ib, et le premier élément d'intrication (7) IIa d'au moins une deuxième paire d'éléments d'intrication IIa-IIb est intriqué avec le deuxième élément d'intrication (9) IIb d'au moins une deuxième paire d'éléments d'intrication IIa-IIb, dans lequel l'intrication quantique de la première paire d'éléments d'intrication Ia-Ib est indépendant de l'intrication quantique d'au moins une deuxième paire d'éléments d'intrication IIa-IIb.

10. Le procédé de transmission d'information d'états quantiques selon la revendication 8 ou 9, dans lequel les paires individuelles d'éléments d'intrication (3, 4, 7, 9) Ia-Ib, IIa-IIb sont constituées d'un matériau qui est un élément chimique de la colonne de bore du tableau périodique ou d'un élément, qui a un nombre impair d'électrons sur la couche de valence d'un atome et qui est sous la forme d'un solide, ou d'un matériau dans lequel il existe des liaisons covalentes entre les atomes, et qui est sous la forme d'un solide.

11. Le procédé de transmission d'informations d'états quantiques selon l'une quelconque des revendications 8 à 10, dans lequel le milieu primaire (2) d'informations quantiques est un solide, un liquide, un gaz, un plasma ou un quelconque mélange de ceux-ci.

12. Le procédé de transmission d'information d'états quantiques selon la revendication 11, dans lequel le milieu primaire (2) d'information quantiques est un monocristal formé à partir d'un élément chimique de la colonne de carbone du tableau périodique.

13. Le procédé de transmission d'information d'états quantiques selon l'une quelconque des revendications 8 à 12, dans lequel le milieu intermédiaire (6) et/ou le milieu terminal (11) est un gaz, un liquide, un solide ou un mélange de ceux-ci.

14. Le procédé de transmission d'information d'états quantiques selon l'une quelconque des revendications 8 à 13, dans lequel le résonateur d'états quantiques (8) et/ou au moins un réservoir terminal (10) est constitué d'un diélectrique polarisé (5).

15. Le procédé de transmission d'information d'états quantiques selon l'une quelconque des revendications 8 à 14, dans lequel le résonateur d'états quantiques (8) et/ou au moins un réservoir terminal (10) est recouvert d'une isolation (12).

Fig. 1

Fig. 2

Fig. 3

Fig. 4

Fig. 5

Fig. 6

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6314189 B1
- CN 108604316 A
- CN 1840486 A

**Non-patent literature cited in the description**

- **BEDNARZ-MISA I ; PIETKIEWICZ J et al.** *Adv.Clin.Exp.Med.,* 2009, vol. 18 (1), 71-78
- **BEDNARZ-MISA I ; PIETKIEWICZ J et al.** *Adv.Clin.Exp.Med,* 2009, vol. 18 (1), 71-78
- *Protein J.,* 2011, vol. 30 (3), 149-158
- **PIETKIEWICZ J. et al.** *Protein J.,* 2011, vol. 30 (3), 149-158